Europäisches Patentamt

European Patent Office

Office européen des brevets

(11) Veröffentlichungsnummer: **0 216 151 B1**

(12) **EUROPÄISCHE PATENTSCHRIFT**

(45) Veröffentlichungstag der Patentschrift: 06.03.91

(21) Anmeldenummer: 86111548.3

(22) Anmeldetag: 20.08.86

(51) Int. Cl.⁵: **C07C 29/17**, C07C 31/125, C07C 45/50, C07C 47/02, C07C 45/82, C07C 45/74, C07C 47/21

(54) Verfahren zur Herstellung von 2-Ethylhexanol.

(30) Priorität: 29.08.85 DE 3530839

(43) Veröffentlichungstag der Anmeldung:
01.04.87 Patentblatt 87/14

(45) Bekanntmachung des Hinweises auf die
Patenterteilung:
06.03.91 Patentblatt 91/10

(84) Benannte Vertragsstaaten:
AT BE CH DE FR GB IT LI LU NL SE

(56) Entgegenhaltungen:
EP-A- 0 052 999
DE-A- 1 913 198
FR-A- 1 563 043
FR-A- 2 314 910
GB-A- 1 158 269

CHEMICAL ABSTRACTS, Band 80, 1974, Seite
326, Ref.Nr. 82068s, Columbus, Ohio, US

HYDROCARBON PROCESSING, Band 59, Nr.
11, 1980, Seite 93-102; B. CORNILS et al.:
"2-EH: What you should know"

(73) Patentinhaber: HOECHST AKTIENGESELL-
SCHAFT
Postfach 80 03 20
W-6230 Frankfurt am Main 80(DE)

(72) Erfinder: Kessen, Gunther, Dr. Dipl.-Chem.
Vennstrasse 6
W-4200 Oberhausen 11(DE)
Erfinder: Cornils, Boy, Dr. Dipl.-Chem.
Kirschgartenstrasse 6
W-6238 Hofheim(DE)
Erfinder: Hibbel, Josef, Dipl.-Ing.
Bruchsteg 13
W-4200 Oberhausen 11(DE)
Erfinder: Bach, Hanswilhelm, Dr. Dipl.-Chem.
Alleestrasse 56
W-4100 Duisburg 11(DE)
Erfinder: Gick, Wilhelm, Dr. Dipl.-Chem.
Im Buschhuck 8
W-4100 Duisburg 74(DE)

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Wiebus, Ernst**
**Ferdinandstrasse 77**
**W-4200 Oberhausen 14(DE)**
Erfinder: **Zgorzelski, Wolfgang, Chem.-Ing.**
**Westmarkstrasse 34**
**W-4200 Oberhausen 11(DE)**

**Beschreibung**

Die vorliegende Erfindung betrifft ein Verfahren zur Herstellung von 2-Ethylhexanol.

Die technische Herstellung von 2-Ethylhexanol erfolgt üblicherweise aus Propen. Durch Hydroformylierung des Olefins erhält man ein n-Butanal enthaltendes Reaktionsgemisch, aus dem durch Destillation reines n-Butanal gewonnen wird. Anschließend aldolisiert man n-Butanal zum entsprechenden Butyraldol, aus dem sich durch Wasserabspaltung bei erhöhter Temperatur 2-Ethylhexenal bildet. In Gegenwart geeigneter Hydrierkatalysatoren wird der ungesättigte Aldehyd zu 2-Ethylhexanol hydriert, das man mittels einer mehrstufigen Destillation reinigt.

2-Ethylhexanol wird in großen Mengen zur Herstellung von Dioctylphthalat, einem bevorzugten Weichmacher für Polyvinylchlorid, verwendet. An den Weichmacher werden erhebliche Anforderungen bezüglich Farbe und Reinheit gestellt. Aus diesem Grunde muß auch das 2-Ethylhexanol hohen Qualitätsanforderungen genügen. Daher ist es erforderlich, bereits das als Einsatzmaterial in die Aldolisierung verwendete n-Butanal in reiner Form zu gewinnen.

Bei der Hydroformylierung von Propen fällt neben dem erwünschten n-Butyraldehyd auch i-Butyraldehyd an. Es ist zwar bekannt, durch Wahl der Reaktionsbedingungen, wie Art und Zusammensetzung des verwendeten Katalysators, Druck, Temperatur und Verweilzeit, die Zusammensetzung des Hydroformylierungsgemisches zu beeinflussen. Es ist aber nicht möglich, die Bildung von i-Butanal gänzlich zu unterbinden.

Das im Hydroformylierungsgemisch stets vorhandene 1-Butanal stört bei der Herstellung von 2-Ethylhexanol, da es selbst in kleinen Mengen dessen Qualität ungünstig beeinflußt. Daher ist es notwendig, das 1-Butanal und alle anderen Nebenprodukte aus dem Hydroformylierungsprodukt zu entfernen, so daß reines n-Butanal als Ausgangsmaterial für die Aldolisierung zur Verfügung steht.

In American Chemical Society Symp. Ser. 1981 (Monohydric Alcohols), 159, S. 71 - 85, ist ein Verfahren zur Herstellung von 2-Ethylhexanol beschrieben. Die Hydroformylierung von Propen verläuft bei relativ niedrigem Druck in Gegenwart eines Rhodium und überschüssiges Triphenylphosphin enthaltenden Katalysators. Das rohe Hydroformylierungsgemisch wird mittels zweier Destillationskolonnen in drei Fraktionen aufgetrennt.

Über Kopf der ersten Kolonne zieht man i-Butyraldehyd und leichter als n-Butanal siedende Stoffe ab. In der zweiten Kolonne trennt man über Kopf reines n-Butanal ab und entfernt mit dem Sumpfprodukt die Höhersieder. Die anschließende Aldolisierung des n-Butanals erfolgt unter Verwendung wäßriger Natronlauge bei Temperaturen von 110 bis 120°C, die Verweilzeit beträgt ungefähr 60 Sek.

Im Verlauf dieser Umsetzung wird aus n-Butanal unter Abspaltung von Wasser 2-Ethylhexenal, das untergeordnete Mengen n-Butanal, trimeres n-Butanal und 2-Ethyl-hexanal enthält, gebildet.

Das in Wasser unlösliche 2-Ethylhexenal wird zusammen mit geringen Mengen Nebenprodukten von der wäßrigen Lauge, die in die Aldolisierung zurückgeführt werden kann, abgetrennt. Anschließend wird das rohe 2-Ethyl-hexenal zu 2-Ethylhexanol hydriert und destillativ gereinigt.

Die bekannten Verfahren lassen eine weitere Erhöhung der Wertproduktausbeute allenfalls in sehr geringem Umfang zu. Dennoch ist man bestrebt, die 2-Ethylhexanol-Ausbeute weiter zu steigern, da 2-Ethylhexanol ein technisches Großprodukt ist und schon kleine Ausbeuteerhöhungen erhebliche wirtschaftliche Vorteile ergeben.

Die bislang unumgängliche Reingewinnung des n-Butanals durch zweistufige Destillation ist mit Nachteilen behaftet. Sie bestehen zum einen in dem hohen Destillationsaufwand und zum anderen in der möglichen Bildung von Höhersiedern, die durch Kondensation aus dem reaktiven n-Butyraldehyd entstehen. Je stärker das Hydroformylierungsprodukt während der destillativen Aufarbeitung thermisch belastet wird, desto höher sind die Verluste an n-Butanal.

Es bestand daher die Aufgabe, ein Verfahren zu entwickeln, das es gestattet, die Abtrennung des für die Aldolkondensation benötigten n-Butanals zu vereinfachen, die Ausbeute an 2-Ethylhexanol zu erhöhen und zugleich die erforderliche hohe Qualität des 2-Ethylhexanols zu gewährleisten.

Diese Aufgabe wird gelöst durch ein Verfahren zur Herstellung von 2-Ethylhexanol durch Umsetzung von Propen mit Kohlenmonoxid und Wasserstoff in Gegenwart eines Rhodium und organische Phosphine enthaltenden Katalysators, Trennung des n-Butanals und i-Butanals, Aldolisierung des n-Butanals zu 2-Ethylhexenal, Hydrierung des 2-Ethylhexenals zu 2-Ethylhexanol und Destillation, dadurch gekennzeichnet, daß Propen in Gegenwart eines Rhodium und sulfonierte Triarylphosphine enthaltenden, in Wasser gelösten Katalysators umgesetzt wird, aus dem Reaktionsgemisch die niedriger als n-Butanal siedenden Anteile destillativ abgetrennt werden, der im wesentlichen aus n-Butanal bestehende Destillationsrückstand in Gegenwart wäßrigen Alkalis aldolisiert und das resultierende 2-Ethylhexenal zunächst in der Gasphase und anschließend in flüssiger Phase an einem fest angeordneten Hydrierkatalysator mit Wasserstoff zu 2-Ethylhexanol umgesetzt wird.

Die Herstellung von n-Butanal erfolgt durch Hydroformylierung von Propen in Gegenwart eines Rhodium und sulfonierte Triarylphosphine enthaltenden, in Wasser gelösten Katalysatorsystems. Diese Umsetzung ist in der Patentschrift DE 26 27 354 C3 beschrieben. Hierbei werden Temperaturen von 50 bis 120 °C angewandt.

Die DE 32 34 701 A1 nennt für die Umsetzung von Olefinen einen Temperaturbereich von 90 bis 150 °C.

In Anlehnung an die in den vorstehend genannten Patentschriften angegebenen Reaktionsbedingungen haben sich bei der Hydroformylierung von Propen Temperaturen von 50 bis 140 °C, insbesondere von 70 bis 130 °C, bevorzugt von 90 bis 125 °C, und Drücke von 0,1 bis 30 MPa, insbesondere von 0,5 bis 10 MPa, bevorzugt von 1 bis 6 MPa, bewährt. Das molare Verhältnis Rhodium zu sulfoniertem Triarylphosphin beträgt 1 : 3 bis 1 : 300, insbesondere 1 : 5 bis 1 : 250, bevorzugt 1 : 10 bis 1 : 200; das Volumverhältnis organische Phase zu Wasserphase liegt bei 1 : 1 bis 1 : 100, insbesondere 1 : 4 bis 1 : 40, bevorzugt 1 : 7 bis 1 : 25.

Nach einer bevorzugten Ausführungsform verwendet man sulfoniertes Triphenylphosphin als sulfoniertes Triarylphosphin.

Aus dem von Kohlenmonoxid, Wasserstoff, Propan und Propen weitgehend befreiten Hydroformylierungsgemisch werden in einer Destillationskolonne mit 100 bis 120 theoretischen Böden sämtliche niedriger als n-Butanal siedenden Bestandteile einschließlich des i-Butanals über Kopf abdestilliert.

Man erholt als Sumpfprodukt ein Gemisch, das neben n-Butanal noch 1 bis 3 Gew.-% (bezogen auf n-Butanal) n-Butanol, i-Butanol, Aldole, die sich von n-Butanal und/oder i-Butanal ableiten, sowie Höhersieder enthält. Es kann ohne weitere Reinigung aldolisiert werden. Dabei verbleiben alle höher als n-Butanal siedenden Verbindungen im n-Butanal.

Die Aldolisierung erfolgt in Gegenwart verdünnter, wäßriger Alkalihydroxidlösungen. Hierbei bildet sich zunächst das entsprechende Butyraldol, aus dem unter Wasserabspaltung 2-Ethylhexenal entsteht. Die Reaktion läuft bei 80 bis 170 °C, insbesondere 90 bis 160 °C, bevorzugt 130 bis 150 °C, ab. Entsprechend der jeweils angewendeten Temperatur stellt sich der Druck ein. Er liegt etwa bei 0,1 bis 0,7 MPa. Die Reaktionszeit beträgt 0,2 bis 5 Min., insbesondere 0,5 bis 4 Min., bevorzugt 1 bis 3 Min. Die wäßrige Alkalihydroxidlösung weist eine Konzentration von 0,5 bis 5 Gew.-%, insbesondere 0,8 bis 4 Gew.-%, bevorzugt 1 bis 2 Gew.-% Alkalihydroxid auf.

Für eine intensive Durchmischung des n-Butanals und der wäßrigen Alkalilösung muß gesorgt werden. Dazu kann beispielsweise ein Rührwerkbehälter oder eine Mischpumpe, der eine Mischstrecke nachgeschaltet ist, Anwendung finden.

Nach der Aldolisierung wird abgekühlt und das gebildete, in Wasser weitgehend unlösliche 2-Ethylhexenal von der wäßrigen, alkalihaltigen Phase abgetrennt. Das rohe 2-Ethylhexenal wird anschließend hydriert. Die abgetrennte, wäßrige Alkaliphase kann in die Aldolisierung zurückgeführt werden. Um den Verbrauch an Alkali zu ergänzen, setzt man gleichzeitig mit cem frischen n-Butanol Alkali, z.B. in Form einer 20 %-igen, wäßrigen Lösung, der Aldolisierungsreaktion zu.

Da eine einstufige Hydrierung des rohen 2-Ethylhexenals weder in flüssiger Phase noch in der Gasphase zu der erforderlichen Produktqualität führt, wird die Hydrierung des rohen 2-Ethylhexenals in zwei aufeinanderfolgenden Stufen durchgeführt. Zunächst wird das 2-Ethylhexenal in der Gasphase, dann in der Flüssigphase hydriert. Die Hydrierung kann in Gegenwart von Lösungsmitteln erfolgen. Geeignete Lösungsmittel sind: Kohlenwasserstoffe, Alkohole oder arteigenes Produkt. Es ist allerdings auch möglich, auf den Einsatz eines Lösungsmittels zu verzichten.

Für die Hydrierung in der Gasphase verwendet man kupferhaltige Katalysatoren, die 40 bis 75 Gew.-%, insbesondere 50 bis 70 Gew.-%, bevorzugt 55 bis 65 Gew.-% Kupfer, bezogen auf die gesamte Katalysatormasse, besitzen. Die Temperaturen liegen bei 110 bis 180 °C, insbesondere 130 bis 170 °C, bevorzugt 140 bis 165 °C. Der Druck stellt sich entsprechend der Temperatur ein.

Als Trägermaterial findet Bimsstein, Aluminiumoxid, Kieselerde, Tonerde und $SiO_2$ in seinen verschiedenen Erschei nungsformen Anwendung. Als Aktivator dienen Erdalkali, Aluminium, Zink und/oder Chrom enthaltende Verbindungen.

Die Raumgeschwindigkeit (flüssiges Produktvolumen: Schüttvolumen des Katalysators x Stunde) beträgt 0,2 bis 0,6, insbesondere 0,3 bis 0,5, bevorzugt 0,35 bis 0,45 je Stunde.

Der Umsatz und Selektivität betragen jeweils mehr als 99%.

Die zweite Hydrierung wird anschließend in flüssiger Phase an einem nickelhaltigen Katalysator durchgeführt. Die Temperaturen liegen bei 100 bis 180 °C, insbesondere 120 bis 140 °C, bevorzugt 125 bis 135 °C. Der Druck beträgt 1 bis 10 MPa, insbesondere 1,5 bis 5 MPa, bevorzugt 2,0 bis 3,0 MPa. Der Katalysator weist 40 bis 70 Gew.-%, insbesondere 45 bis 65 Gew.-%, bevorzugt 55 bis 62 Gew.-% Nickel bezogen auf die gesamte Katalysatormasse, auf. Als Trägermaterial werden Bimsstein, Aluminiumoxid, Kieselerde und Tonerde und $SiO_2$ in seinen verschiedenen Erscheinungsformen verwendet. Als Aktivator dienen Erdalkali, Aluminium, Zink und/oder Chrom enthaltende Verbindungen.

Die Raumgeschwindigkeit (flüssiges Produktvolumen: Schüttvolumen des Katalysators x Stunde) beträgt 0,5 bis 1,5, insbesondere 0,8 bis 1,3, bevorzugt 0,8 bis 1,1 je Stunde.

Umsatz und Selektivität sind nahezu quantitativ.

Das hydrierte Produkt wird destilliert. Das Reinprodukt zeichnet sich durch hervorragende Eigenschaften (gute Farbe und Farbbeständigkeit>, wie für die Herstellung von Weichmachern erforderlich, aus.

Die vorliegende Erfindung wird durch die nachfolgenden Beispiele näher beschrieben:

Hydroformylierung von Propylen

1 Volum-Teil Propylen wird mit 8,75 Volum-Teilen der Rhodium und sulfoniertes Triphenylphosphin enthaltenden wäßrigen Lösung/h (∿ 400 mg Rh/l, 300 g Triphenylphosphintrisulfonat) zusammengebracht.

Las organische Produkt verläßt nach Hydroformylierung das Reaktorsystem und wird von der wäßrigen Katalysatorphase abgetrennt. Die wäßrige Katalysatorlösung wird in die Hydroformylierung zurückgeführt.

$$\begin{array}{lll} \text{Temperatur} & : & 115 \text{ bis } 125 \ ^{\circ}\text{C} \\ \text{Druck} & : & 50 \text{ bar } CO/H_2 = 1:1 \\ \text{Verweilzeit} & : & \sim 30 \text{ Min.} \end{array}$$

Das Rohprodukt enthält

$$\begin{array}{lll} \text{i-}C_4\text{-Aldehyd} & : & 5,0 \text{ Gew.-\%} \\ \text{n-}C_4\text{-Aldehyd} & : & 94,0 \ " \\ \text{Höhersieder} & : & 1,0 \ " \end{array}$$

Destillation des bei der Hydroformylierung anfallenden Roh gemisches:

Das organische Material wird mittels einer Destillationskolonne mit 100 theoretischen Böden aufgetrennt.

```
Druck          :              50 bis 80 kPa
Temperatur : Kopf  :          60 bis 70 °C
                 Sumpf:       90 bis 100 °C
```

Produktzusammensetzung:

a) Kopfprodukt:

```
i-C_4-Aldehyd        99,9 Gew.-%
n-C_4-Aldehyd         0,1 "
```

b) Sumpfprodukt:

```
i-Butanol        0,2 Gew.-%
n-Butanal       98,8      %
```

```
n-Butanol    ⎫
i-Butanol    ⎬    1,0      %
Aldole       ⎪
Höhersieder  ⎭
```

Aldolisierung des n-Butanals

```
1 000 Teile n-Butanal
  10 Teile wäßrige Natronlauge (20 Gew.-%)
   3 Min. Verweilzeit.
```

Aldehyd und Natronlauge werden einer Mischpumpe zugeleitet, dort vermischt und in eine nachgeschaltete Mischstrecke geleitet. Die Temperatur beträgt 130 bis 150 °C. Nach Verlassen der Mischstrecke wird das Produktgemisch abgekühlt und eine Phasentrennung bei etwa 60 °C durchgeführt. Die separierte wäßrige Phase, die etwa 1 bis 2 Gew.-% NaOH aufweist, wird in die Aldolisierung zurückgeführt, während die organische Phase, die mehr als 93 Gew.-% 2-Ethylhexenal enthält, der Hydrierung zugeführt wird.

Hydrierung von 2-Ethylhexenal zu 2-Ethylhexanol

Die Hydrierung des ungesättigten Aldehyds zu 2-Ethylhexanol erfolgt in zwei aufeinander folgenden Schritten: Zunächst gelangt das rohe 2-Ethylhexenal in einen Erhitzer, in dem es verdampft und an einem festangeordneten Hydrierkatalysator hydriert wird.

Im vorliegenden Beispiel wird ein Kupferkatalysator Cu 60/35 (ein Handelsprodukt der Hoechst AG) mit $SiO_2$ als Träger verwendet, der ca. 60 Gew.-% Cu und ca. 10 Gew.-% $SiO_2$ enthält.

Temperatur : 140 bis 160 $^{\circ}C$

Raumgeschwin- : 0,4 (Volumen des flüssigen
digkeit                Produktes/Schüttvolumen
                             Katalysator x Stunde)

Druck : stellt sich entsprechend der
                             gewählten Temperatur ein

Der zweite Hydrierschritt erfolgt zweckmäßigerweise in flüssiger Phase an einem nickelhaltigen Katalysator Ni 55/5, der etwa 55 bis 60 Gew.-% Ni, bezogen auf gesamte Katalysatormasse, aufweist. Als Träger dienen ca. 30 Gew.-% $SiO_2$. Bei diesem Hydrierkatalysator handelt es sich um ein Handelsprodukt der Hoechst AG.

Temperatur : 120 bis 140 $^{\circ}C$
Druck : 2 bis 2,5 MPa
Raumgeschwindigkeit : 0,8 bis 1,1.

Die abschließende Reindestillation erfolgt in bekannter Weise mittels dreier Destillationskolonnen. In der ersten Kolonne werden die niedriger als 2-Ethylhexanol siedenden Komponenten abgetrennt, in der zweiten Kolonne über Kopf reines 2-Ethylhexanol gewonnen, während die dritte Kolonne zur Abtrennung von wiedereinsetzbarem Nachlauf dient.

Die Ausbeute beträgt 97,74 % der Theorie, bezogen auf n-Butanal. Trennt man jedoch das n-Butanal vor Einsatz in der Aldolisierung von den Höhersiedern ab und arbeitet wie in den vorstehenden Beispielen beschrieben, so ergibt sich lediglich eine Ausbeute von 96,77 % der Theorie, bezogen auf n-Butanal.

## Ansprüche

1. Verfahren zur Herstellung von 2-Ethylhexanol durch Umsetzung von Propen mit Kohlenmonoxid und Wasserstoff in Gegenwart eines Rhodium und organische Phosphine enthaltenden Katalysators, Trennung des n-Butanals und i-Butanals, Aldolisierung des n-Butanals zu 2-Ethylhexenal, Hydrierung des 2-Ethylhexenals zu 2-Ethylhexanol und Destillation, dadurch gekennzeichnet, daß Propen in Gegenwart eines Rhodium und sulfonierte Triarylphosphine enthaltenden, in Wasser gelösten Katalysators umgesetzt wird, aus dem Reaktionsgemisch die niedriger als n-Butanal siedenden Anteile destillativ abgetrennt werden, der im wesentlichen aus n-Butanal bestehende Destillationsrückstand in Gegenwart wäßrigen` Alkalis aldolisiert und das resultierende 2-Ethylhexenal zunächst in der Gasphase und anschließend in flüssiger Phase an einem fest angeordneten Hydrierkatalysator mit Wasserstoff zu 2-Ethylhexanol umgesetzt wird.

2. Verfahren nach Anspruch 1, dadurch gekennzeichnet, daß die Umsetzung des Propens mit Kohlenmonoxid und Wasserstoff bei Temperaturen von 50 bis 140 $^{\circ}C$, Drücken von 0,1 MPa bis 30 MPa, einem molaren Ver hältnis Rhodium zu sulfoniertem Triarylphosphin von 1 : 3 bis 1 : 300 und einem Volumverhältnis organische Phase zu Wasserphase von 1 : 1 bis 1 : 100 erfolgt.

## EP 0 216 151 B1

3. Verfahren nach Anspruch 1 und 2, dadurch gekennzeichnet, daß die Abtrennung der niedriger als n-Butanal siedenden Anteile in einer Destillationskolonne mit 100 bis 120 theoretischen Böden erfolgt und das sämtliche höher als n-Butanal sie-dende Anteile enthaltende n-Butanal aus dem Sumpf der Destillationskolonne abgezogen wird.

4. Verfahren nach Anspruch 1 bis 3, dadurch gekennzeichnet, daß die Aldolisierung in Gegenwart wäßriger Lösungen von LiOH, NaOH oder KOH mit 0,5 bis 5 Gew.-% Alkali, bezogen auf die wäßrige Lösung erfolgt, die Temperatur 100 bis 170 °C und die Verweilzeit des n-Butanal-haltigen Destillationsrückstandes 0,2 bis 5 Min. beträgt.

5. Verfahren nach Anspruch 1 bis 4, dadurch gekennzeichnet, daß das Aldolisierungsprodukt 2-Ethyl-hexenal in der Gasphase an einem kupferhaltigen Katalysator bei Temperaturen von 110 bis 180 °C und Drücken von 0,05 bis 0,5 MPa hydriert wird.

6. Verfahren nach Anspruch 5, dadurch gekennzeichnet, daß der kupferhaltige Katalysator 40 bis 75 Gew.-% Kupfer, bezogen auf die gesamte Katalysatormasse, enthält.

7. Verfahren nach Anspruch 1 bis 6, dadurch gekennzeichnet, daß das die Gasphasenhydrierung verlassende Reaktionsgemisch in flüssiger Phase an einem nickelhaltigen Katalysator bei Temperaturen von 100 bis 180 °C und Drücken von 1 bis 10 MPa nachhydriert wird.

8. Verfahren nach Anspruch 7, dadurch gekennzeichnet, daß der für die Hydrierung in flüssiger Phase verwendete Katalysator 40 bis 70 Gew.-% Nickel, bezogen auf die gesamte Katalysatormasse, enthält.

## Claims

1. A process for the production of 2-ethylhexanol by the reaction of propene with carbon monoxide and hydrogen in the presence of a catalyst containing rhodium and organic phosphines, separation of the n-butanal and i-butanal, aldolisation of n-butanal to 2-ethylhexenal, hydrogenation of 2-ethylhexenal to 2-ethyihexanol and distillation, characterised in that propene is reacted in the presence of a catalyst dissolved in water and containing rhodium and sulfonated triarylphosphines , the parts with a boiling point lower than that of n-butanal are separated from the reaction mixture by distillation, the distillation residue mainly consisting of n-butanal is adolised in the presence of aqueous alkali and the resultant 2-ethylhexenal is initially reacted in the gas phase and subsequently in the liquid phase on a rigidly arranged hydrogenation catalyst with hydrogen to 2-ethylhexanol.

2. A process according to claim 1 , characterised in that the reaction of propene with carbon monoxide and hydrogen takes place at temperatures of 50 to 140 °C , pressures of 0.1 MPa to 30 MPa , a molar ratio of rhodium to sulfonated triarylphosphine of 1 : 3 to 1 : 300 and a volume ratio of organic to aqueous phase of 1 : 1 to 1 : 100.

3. A process according to claims 1 and 2, characterised in that the parts with a boiling point lower than that of n-butanal are separated in a distillation column with 100 to 120 theoretical trays and that the n-butanal containing all the parts with a boiling point higher than that of n-butanal is drawn off from the bottom of the distillation column.

4. A process according to claims 1 to 3, characterised in that the aldolisation takes place in the presence of aqueous solutions of LiOH, NaOH or KOH with 0.5 to 5% by weight alkali , the temperature is 100 to 170 °C and the residence period of the distillation residue containing the n-butanal is 0.2 to 5 min.

5. A process according to claims 1 to 4, characterised in that the aldolisation product, 2-ethylhexenal , is hydrogenated in the gas phase on a copper-containing catalyst at temperatures of 110 to 180 °C and pressures of 0.05 to 0.5 MPa.

6. A process according to claim 5, characterised in that the copper-containing catalyst contains 40 to 75% by weight copper, related to the total catalyst mass.

8

7. A process according to claims 1 to 6, characterised in that the reaction mixture leaving the gas phase hydrogenation is post-hydrogenated in liquid phase on a nickel-containing catalyst at temperatures of 100 to 180° C and pressures of 1 to 10 MPa.

8. A process according to claim 7, characterised in that the catalyst used for the hydrogenation in the liquid phase contains 40 to 70% by weight nickel , related to the total catalyst mass.

**Revendications**

1. Procédé pour la fabrication du 2-éthylhexanol par réaction du propène avec le monoxyde de carbone et l'hydrogène en présence d'un catalyseur contenant du rhodium et des phosphines organiques, séparation du n-butanal et du i-butanal, aldolisation du n-butanal en 2-éthylhexénal, hydrogénation du 2-éthylhexénal en 2-éthylhexanol et distillation, caractérisé en ce que L'on fait réagir le propène en présence d'un catalyseur dissous dans l'eau, contenant du rhodium et des triarylphosphines sulfonées, les fractions plus volatiles que le n-butanal sont séparées du mélange réactionnel par distillation, le résidu de distillation, consistant essentiellement en n-butanal, est aldolisé en présence d'alcali aqueux et le 2-éthylhexénal résultant est transformé en 2-éthyl-hexanol par l'hydrogène d'abord en phase gazeuse et ensuite en phase liquide sur un catalyseur d'hydrogénation en lit fixe.

2. Procédé selon la revendication 1, caractérisé en ce que la réaction du propene avec le monoxyde de carbone et l'hydrogène s'effectue à des températures de 50 à 140° C, sous des pressions de 0,1 à 30 MPa, avec un rapport molaire du rhodium à la triarylphosphine sulfonée de 1:3 à 1,300 et un rapport en volume de la phase organique à la phase aqueuse de 1:1 à 1:100.

3. Procédé selon les revendications 1 et 2, caractérisé en ce que la séparation des composés plus volatils que le n-butanal s'effectue dans une colonne à distiller à 100-120 plateaux théoriques et on prélève au pied de la colonne de distillation l'ensemble des composants plus lourds que le n-butanal.

4. Procédé selon les revendications 1 à 3, caractérisé en ce que l'aldolisation est effectuée en présence de solutions aqueuses de LiOH, NaOH ou KOH à 0,5-5 % en poids d'alcali, par rapport à la solution aqueuse, la température est de 100 à 170° C et la durée de passage du résidu de distillation contenant le n-butanal est de 0,2 à 5 min.

5. Procédé selon les revendications 1 à 4, caractérisé en ce que le 2-éthylhexénal du produit d'aldolisation est hydrogéné en phase gazeuse sur un catalyseur contenant du cuivre à des températures de 110 à 180° C et sous des pressions de 0,05 à 0,5 MPa.

6. Procédé selon la revendication 5, caractérisé en ce que le catalyseur contenant du cuivre contient 40 à 75 % en poids de cuivre, par rapport à la masse totale du catalyseur.

7. Procédé selon les revendications 1 à 6, caractérisé en ce que le mélange réactionnel quittant l'hydrogénation en phase gazeuse est encore hydrogéné en phase liquide sur un catalyseur contenant du nickel à des températures de 100 à 180° C et sous des pressions de 1 à 10 MPa.

8. Procédé selon la revendication 7, caractérisé en ce que le catalyseur utilisé pour l'hydrogénation en phase liquide contient 40 à 70 % en poids de nickel, par rapport à la masse totale du catalyseur.